# EUROPÄISCHE PATENTANMELDUNG

(11) **EP 0 702 950 A1**
(43) Veröffentlichungstag der Anmeldung: **27.03.1996**
(21) Anmeldenummer: 95113804.9
(22) Anmeldetag: 02.09.1995
(51) Int. Cl.: A61K 7/48, A61K 31/19

(54) **Verwendung von Fettsäuren gegen Superinfektionen der Haut**

(30) Priorität: 26.09.1994 DE 4434313
(71) Anmelder: Beiersdorf Aktiengesellschaft, D-20245 Hamburg (DE)
(72) Erfinder: Wolf, Florian, Dr., D-20251 Hamburg (DE); Klier, Manfred, Dr., D-21521 Aumühle (DE); Röckl, Manfred, D-22880 Wedel (DE)

(57) **Zusammenfassung**

Verwendung eines wirksamen Gehaltes eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C6 - 20,
(c) unter Verzicht auf Glycerylfettsäureester, ethoxylierte Glycerylfettsäureester und propoxylierte Glycerylfettsäureester
(d) im pH-Bereich unter 8 vorliegend,
als wirksames Prinzip gegen Superinfektionen der Haut.

## Beschreibung

Die vorliegende Erfindung betrifft Wirkstoffe und dermatologische Zubereitungen, solche Wirkstoffe enthaltend, gegen Superinfektionen der Haut.

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Es wurde überraschend gefunden, und darin liegt die Lösung all dieser Aufgaben, daß die Verwendung eines wirksamen Gehaltes eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀,
(c) unter Verzicht auf Glycerylfettsäureester, ethoxylierte Glycerylfettsäureester und propoxylierte Glycerylfettsäureester
(d) im pH-Bereich unter 8 vorliegend,
als wirksames Prinzip gegen Superinfektionen der Haut den Nachteilen des Standes der Technik abhelfen.

Zwar beschreibt die DE-OS 42 29 737 die Verwendung eines wirksamen Gehaltes eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀,
(c) unter Verzicht auf Glycerylfettsäureester, ethoxylierte Glycerylfettsäureester und propoxylierte Glycerylfettsäureester
(d) im pH-Bereich unter 8 vorliegend,
als wirksames Prinzip in kosmetischen Desodorantien, jedoch gibt die angegebene Schrift keinen Hinweis auf Wirksamkeit im Sinne eines Mittels zur Bekämpfung von Superinfektionen der Haut. Die angegebene Schrift lehrt vielmehr in erster Linie die selektive Wirksamkeit solcher Gemische gegen coryneforme Bakterien.

Aus der EP-OS 0 243 145 ist ferner eine antimikrobiell wirksame topische pharmazeutische Zusammensetzung bekannt, die laut den dortigen unabhängigen Ansprüchen 2 und 3 eine ternäre Mischung aus einem Glycerylfettsäureester, bzw. einem ethoxylierten Glycerylfettsäureester und/oder einem propoxylierten Glycerylfettsäureester, einer Mischung aus Fettsäuren und einem pharmazeutisch annehmbaren Träger bekannt, wobei die Fettsäuren aus einer ersten Fettsäure einer Kettenlänge von C₆ ₋ ₁₈ und einer zweiten Fettsäure einer Kettenlänge von C₆ ₋ ₁₈ bestehen.

Die Dodecansäure und andere erfindungsgemäße Fettsäuren werden zwar formal von diesen generischen Fettsäureformeln umfaßt. Die in dieser Schrift beschriebene antimikrobielle Wirkung wird jedoch allein der Wirkung der Glycerylfettsäureester, der ethoxylierten Glycerylfettsäureester bzw. der propoxylierten Glycerylfettsäureester zugeschrieben, welche zwingend allen dort beschriebenen Zusammensetzungen zugrunde liegen.

Diese Schrift weist in eine andere Richtung als die, die durch die vorliegende Erfindung gegeben wird. In überraschender Weise hat sich nämlich herausgestellt, daß ein Zusatz von Glycerylfettsäureestern, ethoxylierten Glycerylfettsäureestern und/oder propoxylierten Glycerylfettsäureestern die antimikrobielle Wirkung des erfindungsgemäßen Gemisches aus Laurinsäure und anderen Fettsäuren erheblich mindert, in manchen Fällen, bei bestimmten Konzentrationen sogar gänzlich aufhebt.

Ferner ist aus der EP-OS 0 465 423 eine pharmazeutische Zusammensetzung zur Bekämpfung von Mikroorganismen bekannt, welche im wesentlichen einen inerten Träger und einen aktiven Bestandteil umfaßt, welcher aus einer wirksamen Menge einer oder mehrerer Verbindungen, gewählt aus Fettsäuren einer Kettenlänge von C₄ ₋ ₁₄ und deren Monoglyceriden sowie der einfach oder mehrfach ungesättigten Fettsäuren einer Kettenlänge von C₁₄ ₋ ₂₂ und deren Monoglyceriden, besteht. Einen Hinweis in die Richtung der vorliegenden Erfindung findet sich in auch dieser Schrift nicht.

Schließlich ist bekannt, Salze der erfindungsgemäßen Säuren in Kombination einzusetzen. Diese sind jedoch nicht erfindungsgemäß aktiv. Die erfindungsgemäßen Gemische entfalten ihre vorteilhafte Wirkung gegen Superinfektionen der Haut nur im pH-Bereich unter 8.

Erfindungsgemäß bevorzugt werden die C₆ ₋ ₂₀-Fettsäuren gewählt aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

Es ist von Vorteil, folgende Gewichtsverhältnisse zu wählen, unabhängig, ob ein Gemisch der Laurinsäure mit einer oder mehreren weiteren Fettsäuren vorliegt:
Laurinsäure : Capronsäure = 50 : 0 bis 50 : 5
und/oder
Laurinsäure : Caprylsäure = 50 : 1 bis 50 : 20
und/oder
Laurinsäure : Pelargonsäure = 50 : 1 bis 50 : 20
und/oder
Laurinsäure : Caprinsäure = 50 : 1 bis 50 : 20
und/oder
Laurinsäure : Myristinsäure = 50 : 5 bis 50 : 30
und/oder
Laurinsäure : Stearinsäure = 50 : 1 bis 50 : 50,
Besonders vorteilhaft ist, folgende Gewichtsverhältnisse zu wählen:
Laurinsäure : Capronsäure = 50 : 0 bis 50 : 1
und/oder
Laurinsäure : Caprylsäure = 50 : 2 bis 50 : 15
und/oder
Laurinsäure : Pelargonsäure = 50 : 2 bis 50 : 15
und/oder
Laurinsäure : Caprinsäure = 50 : 2 bis 50 : 15
und/oder
Laurinsäure : Myristinsäure = 50 : 10 bis 50 : 20
und/oder
Laurinsäure : Stearinsäure = 50 : 2 bis 50 : 20.

Wenn Laurinsäure im Gemisch mit mehreren weiteren Fettsäuren vorliegt, so ist es ferner günstig, ein Gemisch aus Laurinsäure, Caprinsäure und Caprylsäure zu verwenden, besonders, wenn das Gewichtsverhältnis gewählt wird aus dem Bereich
Laurinsäure : Caprylsäure : Caprinsäure = 50 : 1 : 1 bis 50 : 20 : 20.

Bevorzugt wird dann das Gewichtsverhältnis gewählt aus dem Bereich
Laurinsäure : Caprylsäure : Caprinsäure = 50 : 1 : 1 bis 50 : 5 : 5.

Die bevorzugte Ausführungsform der vorliegenden Erfindung besteht darin, das Verhältnis der Laurinsäure zu einer oder auch mehreren der anderen erfindungsgemäßen Fettsäuren so zu wählen, daß es dem Verhältnis in einem hydrierten (gehärteten) Schnitt von Fettsäuren aus natürlichem Kokosfettsäurengemisch entspricht.

Die Zusammensetzung der natürlichen Kokosfettsäuren beträgt etwa:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Ölsäure | 5 - 8 Gew.-% |
| Stearinsäure | 1 - 3 Gew.-% |
| Linolsäure | 0 - 2 Gew.-% |
| Capronsäure | 0 - 1 Gew.-% |

Die Zusammensetzung der gehärteten (hydrierten) Kokosfettsäuren beträgt etwa:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-% |

Laurinsäure (CAS-No. 143-07-7) ist von verschiedenen Anbietern erhältlich, beispielsweise von der Aarhus Oliefabrik A/S, von der Aceto Chemical Company Inc., von der Dansk Sojakagefabrik A/S, von Procter & Gamble Ltd. und anderen.

Hydrierte Kokosnußfettsäure ist unter der Handelsbezeichnung Hydrofol® Acid 631 von der Firma Ashland Chemical Company und unter der Bezeichnung Edenor® HK 8-18 von der Firma Henkel KGaA erhältlich.

Bevorzugt ist folgende Zusammensetzung der erfindungsgemäßen Gemische:

| | |
|---|---|
| Laurinsäure | 1 - 99 Gew.-% |
| Myristinsäure | 0 - 18 Gew.-% |
| Palmitinsäure | 0 - 10 Gew.-% |
| Caprylsäure | 0 - 9 Gew.-% |
| Caprinsäure | 0 - 10 Gew.-% |
| Stearinsäure | 1 - 99 Gew.-% |
| Capronsäure | 0 - 1 Gew.-%, |

jeweils bezogen auf das Gesamtgewicht des Gemisches.

Besonders vorteilhafte Zusammensetzungen werden erhalten, wenn die Zusammensetzung des erfindungsgemäßen Gemisches wie folgt gewählt wird:

| | |
|---|---|
| Laurinsäure | 10 - 90 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 10 - 90 Gew.-% |
| Capronsäure | 0 - 1 Gew.-% |

jeweils bezogen auf das Gesamtgewicht des Gemisches.

Insbesondere werden vorteilhafte Zusammensetzungen erhalten, wenn die Zusammensetzung des erfindungsgemäßen Gemisches wie folgt gewählt wird:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-%, |

jeweils bezogen auf das Gesamtgewicht des Gemisches.

Laut Spezifikation zeichnet sich Edenor® HK 8-18 durch folgenden ungefähren Gehalt an den erfindungsgemäßen Fettsäuren aus:

| | |
|---|---|
| Laurinsäure | 48 Gew.-% |
| Myristinsäure | 18 Gew.-% |
| Palmitinsäure | 8 Gew.-% |
| Caprylsäure | 7 Gew.-% |
| Caprinsäure | 7 Gew.-% |
| Stearinsäure | 10 Gew.-% |
| Capronsäure | 1 Gew.-% |

jeweils bezogen auf das Gesamtgewicht des Gemisches.

Die Verwendung dieses Handelsproduktes ist vorteilhaft.

In den erfindungsgemäßen dermatologischen Zubereitungen beträgt der Gehalt an den erfindungsgemäßen Fettsäuregemischen vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Die erfindungsgemäßen dermatologischen Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die Zubereitungen vorteilhaft in Form von Tinkturen, Shampoos, Wasch-, Dusch- oder Badezubereitungen, Pudern oder Pudersprays vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, welche vorteilhaft als flüssige Zubereitungen mittels einer Roll-on-Vorrichtung auf die gewünschten Hautbereiche aufgetragen werden sollen haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und SorbitanesterEthylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien in Mengen von 0,01 bis 10,0 %, bezogen auf die Gesamt-Zusammensetzung), Suspendiermittel, Puffergemische oder andere übliche kosmetische Grundstoffe beigemischt werden.

Besonders vorteilhaft werden die Antioxidantien gewählt aus der Gruppe bestehend aus
Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α - Carotin, β - Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ - Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α - Hydroxyfettsäuren, Palmitinsäure, Phytinsäure, Lactoferrin), α - Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ - Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin- E - acetat), Vitamin A und Derivate (z.B. Vitamin A - palmitat) sowie Konyferylbenzoat des Benzoeharzes, Flavonen oder Flavonoiden, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Es ist für die vorliegende Erfindung wesentlich, daß der pH-Wert der erfindungsgemäßen, gegen Superinfektionen der Haut wirksamen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Die untere pH-Grenze wird allein von dermatologischen Gegebenheiten bestimmt. Da Dermatika im allgemeinen keinen pH-Wert kleiner als etwa 3,5 - 4 aufweisen sollten, kann dieser Bereich als untere Grenze angesehen werden. Dennoch sind die erfindungsgemäßen Zusammensetzungen grundsätzlich auch bei noch niedrigeren pH-Werten aktiv.
Vorteilhaft wird der pH-Wert der erfindungsgemäßen Formulierungen im sauren bis sehr schwach alkalischen Bereich kleiner als 8 eingestellt, bevorzugt von 4,0 - 7,5 , besonders bevorzugt von 5,0 - 6,5.

Die jeweils einzusetzenden Mengen an Hilfs-, Zusatz- und Trägerstoffen können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Pudersprays eingearbeitet werden, so können die Suspensionsgrundlagen dafür vorteilhaft gewählt werden aus der Gruppe Kieselsäuregele (z.B. solche, die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung.

### Beispiel 1

### Aerosolspray I

(a) Flüssige Phase

| | Gew.-% |
|---|---|
| Octyldodecanol | 0,50 |
| Kokosfettsäureschnitt | 0,50 |
| (enthaltend | |
| Capronsäure 1 % | |
| Caprylsäure 7 % | |
| Caprinsäure 6 % | |
| Laurinsäure 48 % | |
| Myristinsäure 19 % | |
| Stearinsäure 10 %) | |
| Parfüm | q.s. |
| Ethylalkohol | ad 100,00 |

(b) Die unter (a) erhaltene flüssige Phase wird zusammen mit einem Propan/Butan 2,7 - Gemisch im Verhältnis 39 : 61 in Aerosolbehälter abgefüllt.

### Beispiel 2

### Aerosolspray II

(a) Flüssige Phase

| | Gew.-% |
|---|---|
| Octyldodecanol | 0,50 |
| Kokosfettsäureschnitt (Siehe Beispiel 1) | 0,20 |
| Parfüm | q.s. |
| Isopropylalkohol | ad 100,00 |

(b) Die unter (a) erhaltene flüssige Phase wird zusammen mit einem Propan/Butan 2,7 - Gemisch im Verhältnis 39 : 61 in Aerosolbehälter abgefüllt.

### Beispiel 3

### Pumpspray I

| (a) | Gew.-% |
|---|---|
| Ethylalkohol | 60,00 |
| Glycerin | 1,00 |
| PEG-40-Hydriertes Ricinusöl | 2,00 |
| Kokosfettsäureschnitt (Siehe Beispiel 1) | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 4

### Roll on - Gel I

| (a) | Gew.-% |
|---|---|
| 1,3-Butylenglycol | 2,00 |
| Hydroxyethylcellulose (z.B. Tylose 4000, Hoechst) | 0,50 |

| (b) | |
|---|---|
| Wasser | ad 100,00 |

| (c) | |
|---|---|
| Ethylalkohol | 60,00 |
| PEG-40-Hydriertes Ricinusöl | 2,00 |
| Kokosfettsäureschnitt (Siehe Beispiel 1) | 0,30 |
| Parfum | q.s. |

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 5

### Wachsstift I

| | Gew.-% |
|---|---|
| Hydriertes Ricinusöl | 5,00 |
| Bienenwachs | 6,00 |
| Ceresin (Hart-Ozokerit) | 30,00 |
| C₁₂ ₋ ₁₅-Alkyl-Benzoate | 17,00 |
| Kokosfettsäureschnitt (Siehe Beispiel 1) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 6

### Roll on - Emulsion I

| (a) | Gew.-% |
|---|---|
| Tricetearethphosphat | 0,30 |
| Octyldodecanol | 2,00 |
| C₁₂ ₋ ₁₅-Alkyl-Benzoate | 2,00 |
| Kokosfettsäureschnitt (Siehe Beispiel 1) | 0,50 |
| C₁₀ ₋ ₃₀-Alkylacrylate | 0,15 |

| (b) | |
|---|---|
| Wasser | ad 100,00 |
| NaOH | 0,05 |

| (c) | |
|---|---|
| Ethylalkohol | 10,00 |
| Parfum | q.s. |

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

### Beispiel 7

### Aerosolspray IV

(a) Flüssige Phase

| | Gew.-% |
|---|---|
| Octyldodecanol | 0,50 |
| Fettsäuregemisch (Siehe Beispiel 7) | 0,20 |
| Parfüm | q.s. |
| Isopropylalkohol | ad 100,00 |

(b) Die unter (a) erhaltene flüssige Phase wird zusammen mit einem Propan/Butan 2,7 - Gemisch im Verhältnis 39 : 61 in Aerosolbehälter abgefüllt.

### Beispiel 8

### Pumpspray II

| (a) | Gew.-% |
|---|---|
| Ethylalkohol | 60,00 |
| Glycerin | 1,00 |
| PEG-40-Hydriertes Ricinusöl | 2,00 |
| Fettsäuregemisch (Siehe Beispiel 7) | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 9

### Roll on - Gel II

| (a) | Gew.-% |
|---|---|
| 1,3-Butylenglycol | 2,00 |
| Hydroxyethylcellulose (z.B. Tylose 4000, Hoechst) | 0,50 |

| (b) | |
|---|---|
| Wasser | ad 100,00 |

| (c) | |
|---|---|
| Ethylalkohol | 60,00 |
| PEG-40-Hydriertes Ricinusöl | 2,00 |
| Fettsäureschnitt (siehe Beispiel 7) | 0,30 |
| Parfum | q.s. |

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 10

### Wachsstift II

| | Gew.-% |
|---|---|
| Hydriertes Ricinusöl | 5,00 |
| Bienenwachs | 6,00 |
| Ceresin (Hart-Ozokerit) | 30,00 |
| C₁₂ ₋ ₁₅-Alkyl-Benzoate | 17,00 |
| Fettsäuregemisch (siehe Beispiel 7) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 11

### Roll on - Emulsion II

| (a) | Gew.-% |
|---|---|
| Tricetearethphosphat | 0,30 |
| Octyldodecanol | 2,00 |
| C₁₂ ₋ ₁₅ -Alkyl-Benzoate | 2,00 |
| Fettsäuregemisch (siehe Beispiel 7) | 0,50 |
| C₁₀ ₋ ₃₀ -Alkylacrylate | 0,15 |

| (b) | |
|---|---|
| Wasser | ad 100,00 |
| NaOH | 0,05 |

| (c) | |
|---|---|
| Ethylalkohol | 10,00 |
| Parfum | q.s. |

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

### Beispiel 12

### Pumpspray III

| (a) | Gew.-% |
|---|---|
| Ethylalkohol | 60,00 |
| Glycerin | 1,00 |
| PEG-40-Hydriertes Ricinusöl | 2,00 |
| Fettsäuregemisch (siehe Beispiel 7) | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 13

### Roll on - Gel III

| (a) | Gew.-% |
|---|---|
| 1,3-Butylenglycol | 2,00 |
| Hydroxyethylcellulose (z.B. Tylose 4000, Hoechst) | 0,50 |

| (b) | |
|---|---|
| Wasser | ad 100,00 |

| (c) | |
|---|---|
| Ethylalkohol | 60,00 |
| PEG-40-Hydriertes Ricinusöl | 2,00 |
| Fettsäureschnitt (siehe Beispiel 7) | 0,30 |
| Parfum | q.s. |

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 14

### Wachsstift III

| | Gew.-% |
|---|---|
| Hydriertes Ricinusöl | 5,00 |
| Bienenwachs | 6,00 |
| Ceresin (Hart-Ozokerit) | 30,00 |
| C₁₂ ₋₁₅ -Alkyl-Benzoate | 17,00 |
| Fettsäuregemisch (siehe Beispiel 7) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 15

### Roll on - Emulsion III

| (a) | Gew.-% |
|---|---|
| Tricetearethphosphat | 0,30 |
| Octyldodecanol | 2,00 |
| C₁₂ ₋ ₁₅-Alkyl-Benzoate | 2,00 |
| Fettsäuregemisch (siehe Beispiel 7) | 0,50 |
| C₁₀ ₋ ₃₀-Alkylacrylate | 0,15 |

| (b) | |
|---|---|
| Wasser | ad 100,00 |
| NaOH | 0,05 |

| (c) | |
|---|---|
| Ethylalkohol | 10,00 |
| Parfum | q.s. |

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

## Patentansprüche

1. Verwendung eines wirksamen Gehaltes eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C6 - 20,
(c) unter Verzicht auf Glycerylfettsäureester, ethoxylierte Glycerylfettsäureester und propoxylierte Glycerylfettsäureester
(d) im pH-Bereich unter 8 vorliegend,
als wirksames Prinzip gegen Superinfektionen der Haut.

2. Verwendung nach Anspruch 1, dadurch gekennzeichnet, daß die weiteren gesättigte unverzweigte Fettsäuren gewählt werden aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

3. Verwendung nach Anspruch 1, dadurch gekennzeichnet, daß folgende Gewichtsverhältnisse der Fettsäuren untereinander vorliegen:
Laurinsäure : Capronsäure= 50 : 0 bis 50 : 5
und/oder
Laurinsäure : Caprylsäure= 50 : 1 bis 50 : 20
und/oder
Laurinsäure : Pelargonsäure = 50 : 1 bis 50 : 20
und/oder
Laurinsäure : Caprinsäure= 50 : 1 bis 50 : 20
und/oder
Laurinsäure : Myristinsäure = 50 : 5 bis 50 : 30
und/oder
Laurinsäure : Stearinsäure = 50 : 1 bis 50 : 50,
bevorzugt:
Laurinsäure : Capronsäure= 50 : 0 bis 50 : 1
und/oder
Laurinsäure : Caprylsäure= 50 : 2 bis 50 : 15
und/oder
Laurinsäure : Pelargonsäure = 50 : 2 bis 50 : 15
und/oder
Laurinsäure : Caprinsäure= 50 : 2 bis 50 : 15
und/oder
Laurinsäure : Myristinsäure = 50 : 10 bis 50 : 20
und/oder
Laurinsäure : Stearinsäure = 50 : 2 bis 50 : 20.

4. Verwendung nach Anspruch 1, dadurch gekennzeichnet, daß folgende Gewichtsverhältnisse der Fettsäuren untereinander vorliegen:
Laurinsäure : Caprylsäure : Caprinsäure = 50 : 1 : 1 bis 50 : 20 : 20,
bevorzugt:
Laurinsäure : Caprylsäure : Caprinsäure = 50 : 1 : 1 bis 50 : 5 : 5.

5. Verwendung nach Anspruch 1, dadurch gekennzeichnet, daß die Gemische sich zusammensetzen wie folgt:
| | |
|---|---|
| Laurinsäure | 1 - 99 Gew.-% |
| Myristinsäure | 0 - 18 Gew.-% |
| Palmitinsäure | 0 - 10 Gew.-% |
| Caprylsäure | 0 - 9 Gew.-% |
| Caprinsäure | 0 - 10 Gew.-% |
| Stearinsäure | 1 - 99 Gew.-% |
| Capronsäure | 0 - 1 Gew.-%, |
bevorzugt
| | |
|---|---|
| Laurinsäure | 10 - 90 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 10 - 90 Gew.-% |
| Capronsäure | 0 - 1 Gew.-%, |
besonders bevorzugt
| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-%, |
jeweils bezogen auf das Gesamtgewicht des Gemisches.

6. Verwendung nach Anspruch 1, dadurch gekennzeichnet, daß in den Gemischen, das Verhältnis der Fettsäuren zueinander demjenigen entspricht, wie es in einem hydrierten (gehärteten) Schnitt von Fettsäuren aus dem natürlichen Kokosfettsäurengemisch auftritt.

7. Verwendung nach Anspruch 1, dadurch gekennzeichnet, daß die Gemische enthalten:
(a) Laurinsäure und weitere gesättigte unverzweigte Fettsäuren, die in ihrer Zusammensetzung einem hydrierten (gehärteten) Schnitt von Fettsäuren aus dem natürlichen Kokosfettsäurengemisch entsprechen oder
(b) einen solchen Schnitt selbst.

8. Verwendung eines wirksamen Gehaltes eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C6 - 20,
(c) unter Verzicht auf Glycerylfettsäureester, ethoxylierte Glycerylfettsäureester und propoxylierte Glycerylfettsäureester
(d) im pH-Bereich unter 8 vorliegend,
zur Herstellung einer kosmetischen oder dermatologischen Zubereitung gegen Superinfektionen der Haut.
